# EUROPEAN PATENT APPLICATION

(11) **EP 1 803 714 A1**
(43) Date of publication of application: **04.07.2007**
(21) Application number: 05028489.2
(22) Date of filing: 27.12.2005
(51) Int. Cl.: C07D 305/12, A61K 31/337, A61P 3/04

(54) **Process for preparing crystalline forms of orlistat**

(71) Applicant: KRKA, tovarna zdravil, d.d., Novo mesto, 8501 Novo mesto (SI)
(72) Inventor: Simonic, Igor, 8351 Straza pri Novem mestu (SI); Kramar, Andrejka, 8000 Novo mesto (SI); Benkic, Primoz, 1236 Trzin (SI); Stimac, Anton, 1000 Ljubljana (SI); Vajs, Anamarija, 8000 Novo mesto (SI)
(74) Representative: Becker Kurig Straus

(57) **Abstract**

The present invention relates to a process for preparing crystalline forms of orlistat, in particular orlistat crystalline Form I and orlistat crystalline Form II.

## Description

The present invention relates to a process for preparing crystalline forms of orlistat. In particular, the present invention pertains to such a process, wherein a particular temperature profile is applied during the crystallization in an organic solvent.

Orlistat is an agent providing a pancreatic lipase-inhibiting activity and is used for the preparation of medicaments for preventing and treating hyperlipaemia and obesity. Chemical names of Orlistat are (2S,3S,5S)-5-[(S)-2-formamido-4-methylvaleryloxy]-2-hexy-3-hydroxyhexadecanoic acid lactone or N-formyl-L-leucine-[2S-[2alpha(R*),3 beta]]-1-[(3-hexyl -4-oxo-2-oxetanyl)methyl]dodecyl ester. Orlistat exists in several crystalline forms. Analytic data and spectra characterizing the crystalline Form I and II of orlistat can be found in WO 2005/026140.

Syntheses for manufacturing orlistat and a possible precursor thereof, lipstatin, are already known in the art. Examples for suitable methods to manufacture lipstatin and orlistat are described for example in EP 0 129 748, EP 0 189 577, EP 1 028 115, WO 2004/003212, EP 0 803 576, WO 03/048335, EP 1 458 882, WO 2005/007639. Moreover, also several approaches for purifying orlistat have been previously described in the art. These approaches are however associated with significant drawbacks, and for example, provide insufficient yields, provide a product of insufficient purity or are associated with high costs or an insufficient reproducibility.

In particular, WO 2005/026140 describes a process for the preparation of crystalline Form II of orlistat, which comprises preparing a solution of orlistat in one or more ethers; and isolating the crystalline Form II from the solution thereof by the removal of the ether. As well known ether compounds may give rise to chemical hazards, either through the formation of ether peroxides or due to their low boiling point and high inflammability. Therefore, the use of ether as solvent is undesired and should be avoided.

The problem of the present invention is to provide a method which permits the production of different crystalline forms of orlistat in good yields and in high crystalline quality. Additionally, such a method should be highly reproducible and reliable and help to ensure that crystals of the desired defined crystalline form will be obtained.

This problem has been solved by providing a process for preparing orlistat in a crystalline form, said process comprising: providing a fluid comprising orlistat and an organic solvent and having a first temperature; lowering the temperature of said fluid from said first temperature to a second temperature, said second temperature being higher than the saturation temperature of orlistat in said fluid; adding orlistat seeding material having a defined crystalline form in order to obtain a crystallization mixture saturated or oversaturated at said second temperature; lowering the temperature of said crystallization mixture to a final temperature at a cooling rate of not more than 0.2 °C/min, isolating crystalline orlistat having said defined crystalline form.

In the figures,

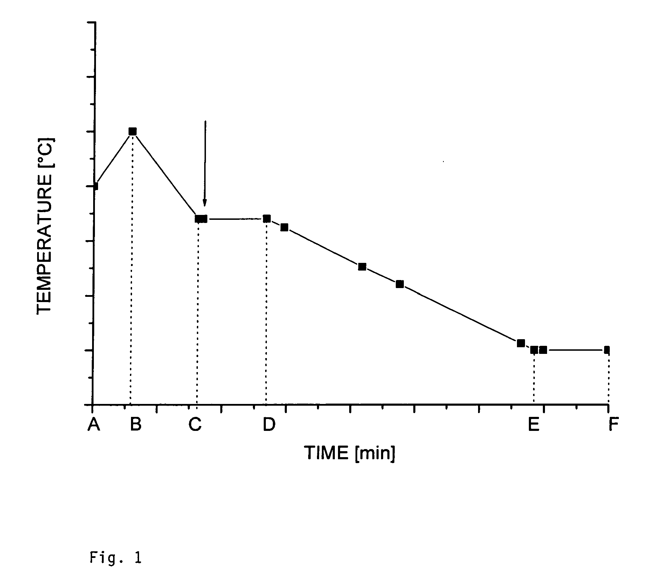

Figure 1 shows a graph describing a crystallization process of the present invention. At time "A" orlistate is added to a solvent, at time "B" the lowering of the temperature of the fluid from a first to a second temperature begins, at time "C" orlisat seeding material is added, at time "D" the lowering to a final temperature begins, at time "E" the final temperature is reached, and the ageing time is from "E" to "F".

The present invention provides a method which permits the production of different crystalline forms of orlistat, in particular of Form I and II of orlistat, via an addition of orlistat seeding material having a defined crystalline form. Additionally, the present invention teaches for the first time that for obtaining crystalline forms of orlistat a specific sequence of heating and cooling steps has to be performed and that the temperature of the crystallization mixture must be lowered at a cooling rate of not more than 0.2 °C/min. When observing the steps of the process according to the present invention, crystalline forms of orlistat of superior quality and in high yields may be obtained. Moreover, the present invention permits to omit an addition of hazardous solvents, such as in particular ethers, or the use of solvents which may not be easily removed from the desired crystalline product, such as water or alcohols, or could give rise to a formation of crystalline forms comprising crystal water.

In the first step of the method according to the present invention a fluid, in particular a solution, is prepared which comprises orlistat and an organic solvent. This fluid can be prepared starting from any of the various forms of orlistat, for example starting from the amorphous or waxy form of orlistat, starting from the crystalline Forms I and II of orlistat, starting from a oily form of orlistat or starting from a crude product comprising orlistat. Orlistat can be prepared according to any desired method, for example according to a method described in EP 0 129 748, EP 0 189 577, EP 1 028 115, WO 2004/003212, EP 0 803 576, WO 03/048335, EP 1 458 882, WO 2005/007639, and in particular by means of a catalytic hydrogenation of lipstatin as described in the Examples of the present invention.

A particular advantage of the present invention is that said process provides the possibility to carry out the preparation of crystalline orlistat in a fluid of orlistat which is free of water or comprises only minor amounts of water remaining present in solvents dried according to standard procedures, such as drying over drying agents or distillation as described in standard laboratory manuals. Similarly, the process of the present invention helps to omit the presence of alcohols which often may not be easily removed during a drying step. In particular, a fluid according to the present invention comprises less than 4 % by weight of water (and/or an alcohol), preferably less than 2 % by weight of water (and/or an alcohol), more preferably less than 1 % by weight of water (and/or an alcohol), most preferably less than 0.5 % by weight of water (and/or an alcohol), each based on the total weight of the fluid, and especially a fluid free of water and/or alcohol.

Advantageously, the fluid comprising orlistat and an organic solvent is a solution. For specific applications, it should be however not excluded that non-dissolved material, in particular in minor amounts, such as e.g. of not more than 1 % by weight (when comparing the weight of the non-dissolved material in dry form to the weight of the dissolved orlistat in dry form) can be present or a clouding may be present in the fluid comprising orlistat and an organic solvent.

This fluid comprising orlistat and an organic solvent is present at a first temperature. In the following step, the temperature of said fluid is lowered to a second temperature, which second temperature is higher than the saturation temperature of orlistat in said fluid, in particular in said solution. The saturation temperature of orlistat in a specific solvent or mixture can be determined on the basis of standard knowledge in the art and on the basis of standard experiments. Moreover, in the context of the present invention, it is considered that a temperature higher than the saturation temperature of orlistat in a particular fluid is present when after holding said fluid for 12 hours without stirring at the respective chosen temperature no spontaneous crystallization and precipitation of orlistat occurs.

Subsequently, orlistat seeding material having a defined crystalline form is added at this second temperature and a solution or fluid saturated or over-saturated with respect to orlistat at said second temperature is obtained. In the context of the present invention, a fluid or solution saturated or over-saturated with respect to orlistat is considered to be present when additional solid orlistat (added e.g. in an amount of 100 mg to a solution or fluid of 1000 ml) remains optically detectable after holding said crystallization mixture for 12 hours without stirring at the respective chosen temperature.

As seeding material in particular orlistat Form I and orlistat Form II may be used. Analytic data and spectra characterizing the crystalline Form I and II of orlistat are known in the art and may be found in WO 2005/026140.

Advantageously, after the addition of the orlistat seeding material, the so obtained crystallization mixture is held for a period of time at said second temperature in order to permit that an ageing of crystals occurs. This additional period of time is advantageous for ensuring high quality and good yields of orlistat according to the present invention. Said additional period of time can take between 1 minute and 1 day, preferably between 10 minutes and 3 hours, more preferably between 15 minutes and 45 minutes.

In the next step, the temperature of the resulting crystallization mixture is lowered at a cooling rate of not more than 0.2 °C/min. A cooling step which is performed at this cooling rate is very important in order to obtain crystalline orlistat of high quality and in good yields and is important for ensuring that orlistat in the defined crystalline form corresponding to the seeding crystal will be obtained.

Particularly good results are obtained when the temperature of said crystallization mixture is lowered at a cooling rate of not more than 0.1 °C/min, preferably wherein the temperature of said crystallization mixture is lowered at a cooling rate of 0.02 to 0.07 °C/min, more preferably wherein the temperature of said crystallization mixture is lowered at a cooling rate of 0.03°C/min. In contrast, an immediate cooling to room temperature, to 0 °C or to an even lower temperature as usually performed in the art after having dissolved orlistat in a solvent, may give rise to devastating results and can give rise to orlistat of an undefined crystalline form, at least in a considerable number of crystallization experiments. The present invention thus largely increases the reliability and reproducibility of the crystallization process.

After having reached the final temperature and optionally having kept the crystallization mixture for an additional period of time allowing an ageing of the crystals (said additional period being for example up to additional 2 days, preferably up to additional 3 hours) at said temperature, the crystals will be separated from the crystallization mixture.

This separation of the orlistat crystals can be obtained by any separation process of the state of the art, for example by means of a filtration or a vacuum filtration. When desired, the crystals can be washed additionally with a small amount of the organic solvent(s) used in the process of the present invention or other appropriate organic solvent(s). In particular, it is advantageous, when the final temperature is in the range of - 30 °C to + 20 °C.

Optionally after the separation and isolation of orlistat a drying step can be performed. This drying step can be performed according to any suitable method known in the art, e.g. a drying procedure "under vacuum", i.e. at a pressure lower than the ambient pressure, and/or a drying procedure at room temperature or at a higher temperature.

The process of the present invention may be in particular of use for obtaining orlistat being in the crystalline Form I of orlistat and crystalline Form II of orlistat. In particular, the present invention permits an access to orlistat in the crystalline Form II in a reproducible manner and in good yields.

Particularly good results are obtained when combining the process of the present invention with an organic solvent which is selected from the group consisting of ketones, hydrocarbons, acetates and mixtures thereof.

For example, the hydrocarbon can be a cyclic, branched or non-branched C5 to C10 alkane or alkene, a non-cyclic, branched or non-branched C5 to C10 alkane or alkene, a C6 to C10 aromatic hydrocarbon. The term "alkene" as used in the context of the present invention indicates a hydrocarbon which contains one, two or more carbon-carbon double bonds. The term "C6 to C10 aromatic hydrocarbon" as used in the present invention indicates a compound comprising an aromatic ring system, such as e.g. a benzene ring or another aromatic ring system, optionally bearing one or more branched or non-branched alkyl or alkenyl side chains, such as e.g. toluene or a xylene, in which compound the total number of the carbon atoms present in the ring and in the side chain(s), when present, is from 6 to 10. Preferred hydrocarbons are hexane, heptane or octane. Particularly good results have been achieved when using heptane or octane, and especially when using heptane.

Advantageous solvents to be used in the present invention are ketones, both cyclic alkyl or alkenyl ketones and non-cyclic alkyl or alkenyl ketones; preferably said ketones are other than acetone.

In case of a non-cyclic ketone R¹-C(O)-R² , the substituent R¹ can be a branched or non-branched C2 to C6 alkyl or alkenyl and R² can be a methyl or a branched or non-branched C2 to C6 alkyl or alkenyl. A cyclic ketone can be for example a cyclic alkyl ketone, such as e.g. cyclohexanone, or a cyclic alkenyl ketone. Said cyclic ketone optionally can be provided with branched or non-branched alkyl side chain(s) and said cyclic ketone has in total 5 to 10 carbon atoms present in the ring and in the side chain(s), when present. Particularly good results have been obtained when using diethyl ketone, a n-propyl ketone, in particular a n-propyl (C1 to C6 alkyl) ketone, an isopropyl ketone, in particular a isopropyl (C1 to C6 alkyl) ketone, isopropyl methyl ketone, di-n-propyl ketone, diisopropyl ketone, n-butyl methyl ketone. Outstanding results have been obtained when using diethyl ketone, an isopropyl (C 1 to C6 alkyl) ketone or a n-propyl (C 1 to C6 alkyl) ketone. The term "(C 1 to C6 alkyl) ketone" means any ketone having an alkyl substituent chosen among C 1 to C6 alkyl.

Moreover, also an acetate (i.e. an ester of acetic acid) can be used as a solvent in a process according to the present invention. Said acetate can be a branched or a non-branched alkyl acetate, wherein said alkyl is a C2 to C6 alkyl, preferably said acetate being a C3 to C4 alkyl, (i.e. a propyl ester of acetic acid or a butyl ester of acetic acid). Particularly good results have been obtained when said acetate being n-propyl acetate, isopropyl acetate, n-butyl acetate.

According to the general knowledge in the art, also mixtures of one or more acetate(s) and/or one or more ketone(s) and/or one or more hydrocarbon(s) can be used in a process according to the present invention.

Additional features and advantages of the present invention are described in and will become apparent from the following, non-limiting examples.

The pharmaceutical composition comprising orlistat in the crystalline Form II can be prepared through the dry or wet granulation procedure. To improve the flowability characteristic of the orlistat different diluents could be used: lactose, mannitol, sorbitol, microcrystalline cellulose and others. Apart from the orlistat and diluents, the composition which is preferably in form of capsule, may also include other excipients and additives conventional in the art, like binders, solubilizing agents, lubricants, glidants and combinations thereof.

### Examples

### Example 1

### Hydrogenation of lipstatin

5.1 g of oily lipstatin (assay 92%) is dissolved in 102 ml of ethanol. The solution is stirred under argon for 5 minutes, then 440 mg of 5% Pd/C is added and the mixture is hydrogenated for 6 hours at ambient temperature and pressure. After hydrogenation, the resulting mixture is filtered and evaporated to the oily substance. 5.03 g of oil which contains 83% of orlistat is obtained.

### Example 2

### Preparation of crystals orlistat

2.78 g of oily orlistat obtained in Experiment 1 is mixed with 11 ml of heptane at room temperature and dissolved by heating to 30°C. The solution is then cooled to 22°C and seeded by 20 mg of orlistat form II. The mixture is then stirred for additional 0.5 hour and cooled in about 2 hours to 10°C. After additional 10 min of stirring, the solid is filtered off and dried in vacuum for 48 hours. 1.5 g (54% yield) of orlistat is obtained. The assay of product is 97%, the form of product is form II confirmed by IR spectra and XR diffractogram.

### Example 3

### Preparation of crystals orlistat

200 g of orlistat of polymorphic form II is mixed with 800 ml of heptane at room temperature and dissolved by heating to 32°C. The solution is then cooled to 22°C and seeded by 1 g of orlistat form II. The mixture is then stirred for 1 hour at 22°C and cooled in about 3 hours to 10°C. The solid is filtered off and dried in vacuum for 24 hours at temp 20 - 25°C. 184 g (92% yield) of orlistat is obtained. The assay of product is 97%, the form of product is form II confirmed by IR spectra and XR diffractogram.

### Example 4

### Preparation of crystals orlistat

15 g of orlistat of polymorphic form I is mixed with 60 ml of heptane at room temperature and dissolved by heating to 30°C. The solution is then cooled to 22°C and seeded by 20 mg of orlistat form II. The mixture is then stirred for 1.5 hour at 22°C and cooled in about 4 hours to 15°C. The solid is filtered off and dried in vacuum for 24 hours at temp 20 - 25°C. 12.6 g (84% yield) of orlistat is obtained. The assay of product is 99%, the form of product is form II confirmed by IR spectra and XR diffractogram.

### Example 5

### Formulation containing orlistat prepared by granulation

| Ingredient | mg/capsule |
|---|---|
| Orlistat | 120.0 |
| Lactose anhydrous | 105.6 |
| Sodium lauryl sulfate | 7.2 |
| Talc | 7.2 |
| Total | 240.0 |

1. Orlistat and lactose anhydrous are mixed in a high share mixer and granulated with the ethanol.
2. Granules are dried at or below 30° C and passed through #18 mesh screen.
3. Sodium lauryl sulphate and talc are added to the granulate and mixed.
4. Granules are filled in a hard gelatine capsule.

In the same example water or the mixture of water and ethanol can be used as a granulation liquid.

### Example 6

### Formulation containing orlistat prepared by dry process

| Ingredient | mg/capsule |
|---|---|
| Orlistat | 120.0 |
| Lactose anhydrous | 57.9 |
| Microcrystalline cellulose | 57.9 |
| Talc | 4.2 |
| Total | 240.0 |

1. Orlistat, lactose anhydrous, microcrystalline cellulose and talc are mixed in a high share mixer.
2. Prepared mixture is filled in a hard gelatine capsule.

## Claims

1. A process for preparing orlistat in a crystalline form, said process comprising:
providing a fluid comprising orlistat and an organic solvent and having a first temperature;
lowering the temperature of said fluid from said first temperature to a second temperature, said second temperature being higher than the saturation temperature of orlistat in said fluid;
adding orlistat seeding material having a defined crystalline form in order to obtain a crystallization mixture saturated or oversaturated at said second temperature;
lowering the temperature of said crystallization mixture to a final temperature at a cooling rate of not more than 0.2 °C/min;
isolating crystalline orlistat having said defined crystalline form.

2. The process according to claim 1, wherein said orlistat in crystalline form is selected from the group comprising crystalline Form I of orlistat and crystalline Form II of orlistat.

3. The process according to any of the preceding claims, wherein said organic solvent is selected from the group consisting of ketones, hydrocarbons, acetates and mixtures thereof.

4. The process according to any of the preceding claims, wherein the temperature of said crystallization mixture is lowered at a cooling rate of not more than 0.2 °C/min, preferably wherein the temperature of said crystallization mixture is lowered at a cooling rate of 0.02 to 0.07 °C/min, more preferably wherein the temperature of said crystallization mixture is lowered at a cooling rate of 0.03°C/min.

5. The process according to any of the preceding claims, wherein said orlistat in crystalline form is crystalline Form II orlistat.

6. The process according to claim 3, wherein said hydrocarbon is a cyclic, branched or non-branched C5 to C10 alkane or C5 to C 10 alkene, a non-cyclic, branched or non-branched C5 to C10 alkane or C5 to C10 alkene, an C6 to C10 aromatic hydrocarbon, preferably said hydrocarbon being hexane, heptane or octane, more preferably heptane or octane, most preferably heptane.

7. The process according to claim 3, wherein said ketone is a non-cyclic ketone R¹-C(O)-R², wherein R¹ is a branched or non-branched C2 to C6 alkyl or C2 to C6 alkenyl and R² is methyl or a branched or non-branched C2 to C6 alkyl or C2 to C6 alkenyl, or said ketone is a cyclic branched or non-branched alkyl ketone or a cyclic branched or non-branched alkenyl ketone, preferably said ketone being a diethyl ketone, a n-propyl ketone, in particular n-propyl (C1 to C5 alkyl) ketone, a isopropyl ketone, in particular isopropyl (C2 to C5 alkyl) ketone, an isopropyl methyl ketone, din-propyl ketone, diisopropyl ketone, n-butyl methyl ketone, more preferably said ketone being diethyl ketone, a isopropyl ketone, a n-propyl ketone.

8. The process according to claim 3, wherein said acetate is a branched or non-branched alkyl acetate, wherein said alkyl is a C2 to C6 alkyl, preferably said acetate being a C3 to C4 alkyl, more preferably said acetate being n-propyl acetate, isopropyl acetate, n-butyl acetate.

9. The process according to any of the preceding claims, wherein the fmal temperature is in the range of - 30 °C to 20 °C.

10. Orlistat granulation formulation, said formulation comprising granules of lactose and orlistat as prepared according to a process of any of the claims 1 to 9, and optionally further lauryl sulphate and talc.

11. A process for preparing an orlistat granulation formulation, said process comprising mixing orlistat prepared by a process according to any of the claims 1 to 9 and lactose;
granulating orlistat and lactose with an alcohol and/or water;
drying the resulting granules; and
passing said granules through a mesh screen;
said process optionally further comprising:
adding lauryl sulphate and talc to the granulate and mixing lauryl sulphate, talc and granulate; and/or
filling said granules in an appropriate carrier.
